# EUROPEAN PATENT SPECIFICATION

(11) **EP 2 188 629 B1**
(45) Date of publication and mention of the grant of the patent: **14.09.2011**
(21) Application number: 08786447.6
(22) Date of filing: 25.07.2008
(51) Int. Cl.: G01N 33/50, G01N 33/569

(54) **NATIVE GP41 ASSAY**
NATIVES GP41-TESTVERFAHREN
TEST GP41 NATIF

(30) Priority: 26.07.2007 EP 07113185
(43) Date of publication of application: 26.05.2010
(73) Proprietor: Tibotec Pharmaceuticals, Co Cork (IE)
(72) Inventor: VAN ACKER, Koenraad, Lodewijk, August, B-9140 Temse (BE); MEERSSEMAN, Geert Henri, B-1000 Brussels (BE); DAMS, Géry Karel Julia, B-3583 Paal-Beringen (BE); OHAGEN, Asa Catrine, Glasgow G1 1EJ (GB); BUNKENS, Lieve Elisabeth Louis, 3120 Tremelo (BE); HOLEMANS, Pascale Alice Jan, B-2590 Berlaar (BE)
(74) Representative: Daelemans, Frank F.R.
(86) International application number: PCT/EP2008/059786
(87) International publication number: WO 2009/013352

(56) References cited:
- WO-A-03/027255
- US-A1- 2002 077 284
- LIU, SHUWEN ET AL: "Rapid and automated fluorescence-linked immunosorbent assay for high-throughput screening of HIV-1 fusion inhibitors trageting gp41" JOURNAL OF BIOMOLECULAR SCREENING, vol. 8, no. 6, 2003, pages 685-693, XP009091409

### SEQUENCE LISTING

<110> Tibotec Pharmaceuticals Ltd.
<120> Native gp41 assay
<130> TIP 175 PCT
<150> 07113185.8
   <151> 2007-07-26
<160> 2
<170> PatentIn version 3.3
<210> 1
   <211> 34
   <212> PRT
   <213> Human immunodeficiency virus type 1
<400> 1
   Trp Met Glu Trp Asp Arg Glu Ile Asn Asn Tyr Thr Ser Leu Ile His
   1               5                   10                  15
   Ser Leu Ile Glu Glu Ser Gln Asn Gln Gln Glu Lys Asn Glu Gln Glu
               20                  25                  30
   Leu Leu
<210> 2
   <211> 6
   <212> PRT
   <213> Linker
<400> 2
   Gly Ser Ser Gly Gly Lys
   1               5

## Description

The present invention is directed to a method for identifying compounds that decrease the ability of Human Immunodeficiency Virus (HIV) to enter previously uninfected cells.

Entry of HIV into a host cell is a multi-step process, with the viral envelope glycoprotein Env playing a pivotal role. Env is a trimeric glycoprotein complex consisting of surface gp120 subunits non-covalently bound to the transmembrane gp41 subunits that anchor the Env spikes in the viral envelope. Apart from the transmembrane region, the molecular sequence of gp41 includes a fusion domain and two helical "heptad-repeat" regions (HR1 and HR2).

In a first step of the HIV-1 entry process, the gp120 moiety docks in the cellular CD4 receptor on the host-cell surface. This induces conformational changes in gp120 resulting in binding of gp120 to the cellular chemokine co-receptor and insertion of the N-terminal fusion domain of gp41 in the target cell membrane. This relatively stable conformation is called the pre-hairpin intermediate.

Next, the HR2 regions collapse into the hydrophobic grooves of an interior coiled-coil formed by the corresponding homotrimeric HR1 regions, resulting in a stable trimer-of-hairpins, the six-helix bundle. Ultimately, the conformational changes of gp120 and gp41 mediated by the cellular receptors / co-receptors brings the viral and cell membranes into close proximity for membrane fusion and subsequent viral entry. Inhibition of the six helix bundle formation of HR1 and HR2, e.g. in the hairpin conformation, is an attractive target for medicinal intervention.

CD4 (cluster of differentiation 4) is a glycoprotein expressed on the surface of T helper cells, regulatory T cells, monocytes, macrophages, and dendritic cells. It was discovered in the late 1970s and was originally known as leu-3 and T4 (after the OKT4 monoclonal antibody that reacted with it) before being named CD4 in 1984.

The CD nomenclature was proposed and established in the 1st International Workshop and Conference on Human Leukocyte Differentiation Antigens (HLDA), which was held in Paris in 1982. This system was intended for the classification of the many monoclonal antibodies (mAbs) generated by different laboratories around the world against epitopes on the surface molecules of leukocytes (white blood cells). Since then, its use has expanded to many other cell types, and more than 320 CD unique clusters and subclusters have been identified. The proposed surface molecule is assigned a CD number once two specific monoclonal antibodies (mAb) are shown to bind to the molecule.

Two commonly used CD molecules are CD4 and CD8, which are generally used as markers for helper and cytotoxic T cells, respectively. When defining T cells, these molecules are defined in combination with CD3+, as some other leukocytes also express these CD molecules (some macrophages express low levels of CD4, dendritic cells express high levels of CD8). CD4 is also an essential receptor during HIV infection, allowing the HIV to bind to the helper T cell and destruction of CD4+ T cells. The relative abundance of CD4+ and CD8+ T cells is often used to monitor the progression of an HIV infection.

Assays for gp41 inhibitors are most commonly based on peptides representing the two heptad repeat regions. These assays are reductionistic by nature and the true structure of the pre-fusion gp41 is unknown. The viral gp41 proteins are forming trimers complexed to gp120 proteins. This functional HIV-1 Env complex is flexible and can adopt different prefusion states depending on their engagement with cellular receptors. The unliganded Env state is only known from low-resolution electron microscopic data and the atomic details are unclear.

Indeed, in the unliganded spikes it is unknown how the outer gp120 keeps the membrane-anchored gp41 in a meta-stable condition.

For CD4-bound Env complexes, the situation is somewhat better with crystallographic structures of the core of the gp120 protein (Huang et al, 2005, Science, 310: 1025-1028). Importantly, there is no direct structural information describing complete trimeric spikes. The conformation of gp41 in relation to gp120 is unknown but the current assumption is that most of gp41 is contained in a "stalk" that is shielded by a tri-lobed "head" (gp120) region. This simplified model does not explain the mechanism of entry nor fits with experimental data on entry inhibition.

This shows the need for a physiologically relevant model system that is not based on unsupported assumption about the structure of Env. Intuitively, the viral Env as expressed on the surface of an infected cell or a virus should be the most relevant model system.

The assay in accordance with the invention is based on the binding of an exogenously added HR2 derived peptide to a CD4-induced Env protein (liganded spike) and its interference by molecules or so-called candidate compounds.

Said candidate compounds are either chemical molecules or peptides or a combination of both. Specifically, persistently HIV-1 IIIB infected cells are incubated with a soluble form of CD4 (sCD4) prior to exposure to candidate compounds and a labeled HR2-derived peptide (preferably C34-FITC).

The assay protocol according to the invention includes two dispensing manipulations but no wash steps. HUTIIIB cells are incubated 37°C or at room temperature, preferably at room temperature, in the presence of sCD4 at a concentration of 250nM. During this step, gp120 undergoes conformational changes such that increased binding of gp41-derived biological reagents is observed.

In the next step, the activated HUTIIIB cells are added to 96 well plates with or without candidate test compounds. During this incubation at room temperature said compounds or inhibitors are allowed to bind to their target for a brief period before the labeled C34 peptide does.

The second addition includes C34-FITC at a prefered concentration of 250 pM.

The assay end-point is detected by flow cytometry at a speed of 45 minutes per 96 well plate. Thus, the assay is performed using U-bottom plates that are compatible with flow cytometry. Notably, it is not necessary to wash off unbound C34-FITC before read out, making this a homogeneous cell based binding assay. Thus, sCD4 induced complexation of C34-FITC to liganded spikes is measured in solution.

The assay was initially developed for flow cytometric based read-out and was later transitioned onto a high content screening system (Opera^{™}, Evotec Technologies).

The current invention concerns a method for measuring the interference of candidate compounds interfering in the formation of a complex between a C34 peptide derived of the heptad-repeat 2 (HR2) region of Human Immunodeficiency Virus (HIV) and the corresponding heptad-repeat 1 region of gp41 after induction with a human cellular soluble CD4 (sCD4) comprising:
contact cells, expressing native HIV-1 envelope spikes containing gp41, with human sCD4 to obtain sCD4 induced cells,
contact said induced cells with a candidate compound,
provide, optionally together with said candidate compound, a labeled C34 peptide of HIV-1 consisting essentially of the amino acid sequence SEQ ID NO:1 and measure the interference of said compound with the complex formation between HR1 in the native spike and exogenously added labeled C34 by determining the % inhibition as a consequence of the presence or absence of said candidate compound respectively.

Alternatively, measuring the interference of candidate compounds interfering in the formation of a complex between a C34 peptide derived of the heptad-repeat 2 (HR2) region of Human Immunodeficiency Virus (HIV) and the corresponding heptad-repeat 1 region of gp41 after induction with a human cellular soluble CD4 (sCD4) comprises:
contact cells, expressing native HIV-1 envelope spikes containing gp41, with a candidate compound,
contact said cells with sCD4 to obtain sCD4 induced cells,
provide, optionally together with said sCD4, a labeled C34 peptide of HIV-1 consisting essentially of the amino acid sequence SEQ ID NO:1 and
measure the interference of said compound with the complex formation between HR1 in the native spike and exogenously added labeled C34 by determining the % inhibition as a consequence of the presence or absence of said candidate compound respectively.

In a further embodiment of the invention measuring the interference of candidate compounds interfering in the formation of a complex between a C34 peptide derived of the heptad-repeat 2 (HR2) region of Human Immunodeficiency Virus (HIV) and the corresponding heptad-repeat 1 region of gp41 after induction with a human cellular soluble CD4 (sCD4) comprises:
contact cells, expressing native HIV-1 envelope spikes containing gp41, with a candidate compound together with sCD4 to obtain sCD4 induced cells,
provide a labeled C34 peptide of HIV-1 consisting essentially of the amino acid sequence SEQ ID NO:1 and
measure the interference of said compound with the complex formation between HR1 in the native spike and exogenously added labeled C34 by determining the % inhibition as a consequence of the presence or absence of said candidate compound respectively.

The cells, expressing native HIV-1 envelope spikes containing gp41, are preferably HIV-infected cells.

More preferably, the HIV-infected cells used in above mentioned method are IIIB-infected HUT78 cells whereafter binding of labeled C34 to the sCD4 induced spikes is measured by flow cytometry or microscopic methods.

The label used in the method according to the invention is an enzyme, green fluorescent protein or a mutant thereof, fluorescent substance, chemiluminescent substance or radioisotope or combinations thereof.

Preferably the label used is a fluorescent substance like FITC. Said FITC is linked to peptides or polypeptides (proteins) with binding capacity to sCD4 induced HIV-1 spikes. An example given is the C34 peptide derived from HR2 e.g. having the amino acid sequence WMEWDREINNYTSLIHSLIEESQNQQEKNEQELL (SEQ ID NO: 1). Peptide C34 can be labeled either at the N- or C-terminal end and can be shorter in length as well. The C34 peptide can also be embedded in a longer sequence for the purpose of the invention.

The C34 peptide may also contain one or more insertion, substitution or deletion of an amino acid.

The FITC label can be linked to the C34 peptide by a linker with amino acid sequence GSSGGK (SEQ ID NO: 2). Alternatively, FITC is directly coupled to the N terminal, C terminal or internal residue of the peptide.

FITC (fluorescein isothiocyanate) is one of the well-known fluorescein derivatives.

Fluorescein is a fluorophore commonly used in microscopy, in a type of dye laser as the gain medium, in forensics and serology to detect latent blood stains, and in dye tracing. Fluorescein has an absorption maximum at 494 nm and emission maximum of 521 nm (in water). Also, fluorescein has an isoabsorptive point (equal absorption for all pH values) at 460 nm. Fluorescein is also known as a color additive (D&C Yellow no. 7). The disodium salt form of fluorescein is known as D&C Yellow no. 8.

FITC is the original fluorescein molecule functionalized with an isothiocyanate group (-N=C=S), replacing a hydrogen atom on the bottom ring of the structure. This derivative is reactive towards amine groups on proteins inside cells. A succinimidyl-ester functional group attached to the fluorescein core, creating NHS-fluorescein, forms another common amine reactive derivative.

Other derivatives of fluorescein which can be used in the method accoring to the invention include but not restricted to Oregon Green, Tokyo Green, SNAFL, and carboxynaphthofluorescein. These derivatives, along with newer fluors such as Alexa 488 and DyLight 488, have been tailored are preferably used where higher photostability, different spectral characteristics, or different attachment groups are needed.

Measurement in accordance with the current invention of the interference of the candidate compound in the formation of a complex between a C34 peptide of the heptad-repeat 2 (HR2) region of Human Immunodeficiency Virus (HIV) and the corresponding heptad-repeat 1 region of gp41 after induction with a human cellular soluble CD4 (sCD4) is performed by flow cytometry, fluorescence microscopy, fluorimetry, enzyme immunoassay, radiolabelling or chemiluminiscent techniques.

Part of the invention is also the screening for compounds that inhibit the interaction between HR1 and HR2 of HIV-1 and subsequently viral entry in host cells. The inventive homogeneous competitive cell-based binding assay is used for this purpose. The homogeneous competitive cell-based binding assay uses thereto persistently HIV-1 infected cells, which expresses envelope spikes containing gp41. These cells were exposed to soluble CD4 (sCD4) and a fluorescent peptide fragment of HR2 (C34-FITC). Soluble CD4 surprisingly induces conformational changes in the native spike and allows binding of C34-FITC to the HR1 region of gp41, which fluorescently stains the cell membrane in a dotted pattern.

The cell-based assay used is homogenous which means that no wash steps are necessary to separate C34-FITC from the spike expressing cells.

The compound identified using the procedure of the invention as described in this application is thereafter formulated in a pharmaceutically acceptable form.

Alternatively the invention also relates to a method for the production of a pharmaceutical composition comprising the method disclosed above and furthermore mixing the compound identified or a derivative or homologue thereof with a pharmaceutically acceptable carrier.

Part of the invention is also the use of a compound as identified by the method of the invention to inhibit or prevent the membrane fusion process of Human Immunodeficiency Virus (HIV) with the cellular membrane of human cells.

Sequences disclosed in the current description are:
SEQ ID NO: 1
   WMEWDREINNYTSLIHSLIEESQNQQEKNEQELL
SEQ ID NO: 2
   GSSGGK

### Examples

### Example 1: Development of a HIV-1 expressing cell line

The assay requires cells exhibiting high expression levels of functional Env complexes. Therefore, a HUT78 cell line was infected with IIIB virus (HUTIIIB bulk) and cloned to have higher env expression levels (HUTIIIB clone 199-76), as visualized by FACS analysis after anti-gp120 staining. The high-expressing cell clone 199-76 is referred to as HUTIIIB in these examples.

The released virus from the Env-expressing cells was sequenced to assure that no functionally important residues occurred during the cell cloning. The virus from the 199-76 cell clone exhibited three changes in Env as compared to the reference IIIB sequence: R166G (in gp120 V2), E268K (in gp120 C2) and V708A (in gp41 cytoplasmic tail). None of these changes appeared to be critical for the assay set-up or the functionality of the Env protein.

### Example 2: sCD4 induced binding of C34-FITC to HUTIIIB env complexes

The HR2-derived peptide used in this assay is C34 (WMEWDREINNYTSLIHSLIEESQNQQEKNEQELL) labeled with FITC via a GS5 linker (amino acid sequence: GSSGGK), although the sCD4 induced binding of C34-FITC without linker was also demonstrated.

Conceptually, C34 can be labeled either at the N- or C-terminal or at an internal residue of the peptide. The alternative labeling sites were evaluated by binding studies on sCD4 induced/non-induced cells. The C-terminally labeled C34 showed an increased binding to HUTIIIB cells after sCD4 induction. N-terminally labeled C34 also showed an increased binding after sCD4 induction, but lower compared to the C-terminally labeled peptide. Neither of the labeled peptides showed sCD4 induced binding to uninfected HUT78 cells. These data show that the C-terminally labeled C34 appeared to have a more physiologically relevant binding mode. This peptide was selected for further assay optimization.

Titration experiments with sCD4 and C34-FITC indicated that a combination of 250 nM sCD4 and 0.25 nM C34-FITC gave the most robust signal for FACS-based read-out in the absence of cytotoxicity (Z' = 0.81).

| **SCD4 Induced HutIIIB without C34FITC** | | | | | |
|---|---|---|---|---|---|
| 3.55 | 3.52 | 3.59 | 3.52 | 3.55 | 3.52 |
| 3.59 | 3.59 | 3.52 | 3.55 | 3.59 | 3.59 |
| 3.52 | 3.59 | 3.55 | 3.55 | 3.55 | 3.55 |
| 3.55 | 3.49 | 3.55 | 3.62 | 3.55 | 3.59 |
| 3.59 | 3.59 | 3.52 | 3.55 | 3.59 | 3.55 |
| 3.55 | 3.59 | 3.55 | 3.55 | 3.49 | 3.62 |
| 3.52 | 3.59 | 3.52 | 3.52 | 3.52 | 3.55 |
| 3.59 | 3.62 | 3.55 | 3.59 | 3.55 | 3.62 |

| **SCD4 Induced HutIIIB with C34FITC** | | | | | |
|---|---|---|---|---|---|
| 6.32 | 6.21 | 6.15 | 6.04 | 6.10 | 6.49 |
| 6.44 | 6.61 | 6.32 | 6.26 | 6.38 | 6.55 |
| 6.49 | 6.55 | 6.15 | 6.26 | 6.32 | 6.49 |
| 6.38 | 6.49 | 6.32 | 6.38 | 6.38 | 6.38 |
| 6.44 | 6.55 | 6.38 | 6.32 | 6.15 | 6.79 |
| 6.26 | 6.26 | 6.26 | 6.26 | 6.44 | 6.26 |
| 6.49 | 6.44 | 6.21 | 6.38 | 6.32 | 6.44 |
| 6.44 | 6.44 | 6.26 | 6.38 | 6.44 | 6.26 |

| | | | | | |
|---|---|---|---|---|---|
| Half of a 96 well U-bottom plate was filled with positive controls (HUTIIIB cells with C34-FITC) and the other half with negative controls (HUTIIIB cells without C34-FITC). The Z' factor was determined using the following formula (Zhang *et al.*, 1996): Z'= 1-((3*SD pc + 3 * SD ng)/ABS (MEAN pc - MEAN nc). pc: positive control; nc: negative control. | | | | | |

### Example 3: inhibition of C34-FITC binding to env complexes by peptides

To assess the specificity of the C34-FITC binding to the env complex a panel of peptides was tested (Table 1). The specificity of three HIV specific fusion inhibiting peptides (T20, T1249 and C34), one RSV specific fusion inhibiting peptide (C39) and one antibody which acts as a CD4-gp120 inhibitor (F105) was demonstrated by addition of these compounds simultaneously with C34-FITC, but after sCD4 induction.

**Table 1. Peptides tested during specificity experiments.**

| Target | Name | EC50 (µM) |
|---|---|---|
| HIV entry fusion | T20 | = 0.032 |
| HIV entry fusion | C34 | = 0.003 |
| HIV entry fusion | T1249 | = 0.012 |
| RSV entry fusion | RSV-C39 | >20 |
| HIV CD4-gp120 | F105 | >0.10 |

Peptide C34, identical to the labeled peptide, was a more potent inhibitor than T20, which only partially overlaps with C34. T1249 has characteristics of both C34 and T20 and is expected to have an activity between C34 and T20. This was confirmed by the assay results. The irrelevant peptide C39 was inactive, while the F105 antibody potentially inhibits the induction step of the assay, as the epitope of the F105 antibody partially overlaps the binding site of sCD4. However, when F105 is added after sCD4 induction no fusion inhibition of F105 is observed (Table 1), indicating that F105 interferes with sCD4 induction step and not with fusion.

### Example 4: binding of mutant C34-FITC peptides to env complexes

A series of unlabeled C34 mutant peptides were made with changes at predicted and reported interaction points between HR2 and HR1. Introduced mutations have a clear effect on the inhibition potency of the mutant peptides, supporting the specificity of sCD4 induced C34-FITC binding. Wild-type C34 is between 10 and 30.000 times more potent compared with the tested mutant peptides (Table 2).

**Table 2. Activities of wt and mutant C34 peptides in native gp41 assay.**

| | Native gp41 IC50 (µM) |
|---|---|
| HIV-C34 | 0.00049 |
| HIV-C34 628 (W-A) | 0.0053 |
| HIV-C34 631 (W-A) | 0.19 |
| HIV-C34 645 (L-A) | 0.0067 |
| HIV-C34 646 (I-A) | 0.44 |
| HIV-C34 628 (W-A) 631 (W-A) | 1.2 |
| HIV-C34 645 (L-A) 646 (I-A) | 15 |
| HIV-C34 655 (K-A) 656 (N-A) 657 (E-A) | 1.5 |

### Example 5: inhibition of C34-FITC binding to env complexes by small molecules

Although ATA did not generate an EC50 value, there was some non-specific inhibition (about 40% inhibition) in this assay (Table 3). In addition, a panel of other non-specific inhibitors (DS5000) or inhibitors of other HIV targets showed no activity. Of all tested small molecules, only ADS-J showed an inhibition activity above 50%.

**Table 3. Small molecules tested during specificity experiments.**

| Target | Name | Native gp41 EC50 (µM) |
|---|---|---|
| Non-specific | ADSJ | = 0.60 |
| Non-specific | ATA | >200 |
| HIV coreceptor X4 | AMD3100 | >200 |
| HIV CD4-gp120 | BMS-806 | >200 |
| HIV NNRTI | EFV | >200 |
| HIV protease | SQV | >200 |
| HIV RT | AZT | >200 |
| HIV integrase | L870,810 | >200 |
| Non-specific | DS5000 | >200 |
| HIV coreceptor R5 | Maraviroc | >200 |

### Example 6: sequence adding of compounds and sCD4

Addition of compound at different time points was tested by adding the compounds to the HUTIIIB cells either before, after and during sCD4 induction. The tested compounds were C34, T20, T1249, and BMS-806.
- Addition of compound after sCD4 induction
   Cells + sCD4
   Incubation 30' at RT
   Add compound and C34-FITC
   Incubation 2 h at RT
   Read-out
- Addition of compound before sCD4 induction
   Cells + compound
   Incubation 30' at RT
   sCD4 induction (30' at RT)
   add C34-FITC
   Incubation 2 h at RT
   Read-out
- Addition of compound together with sCD4 induction
   Cells + compound + sCD4
   Incubation 30' at RT
   add C34-FITC
   Incubation 2 h at RT
   Read-out

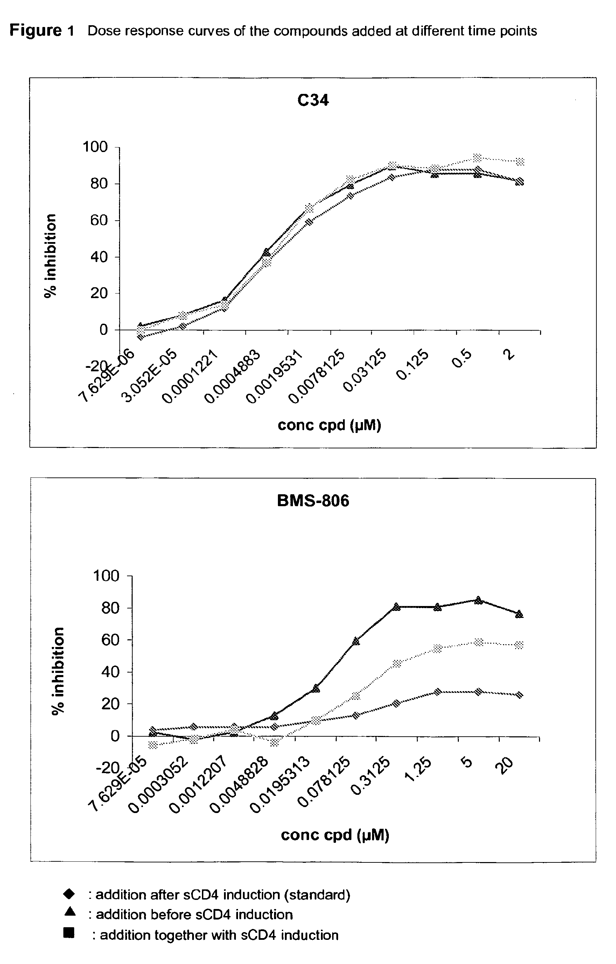

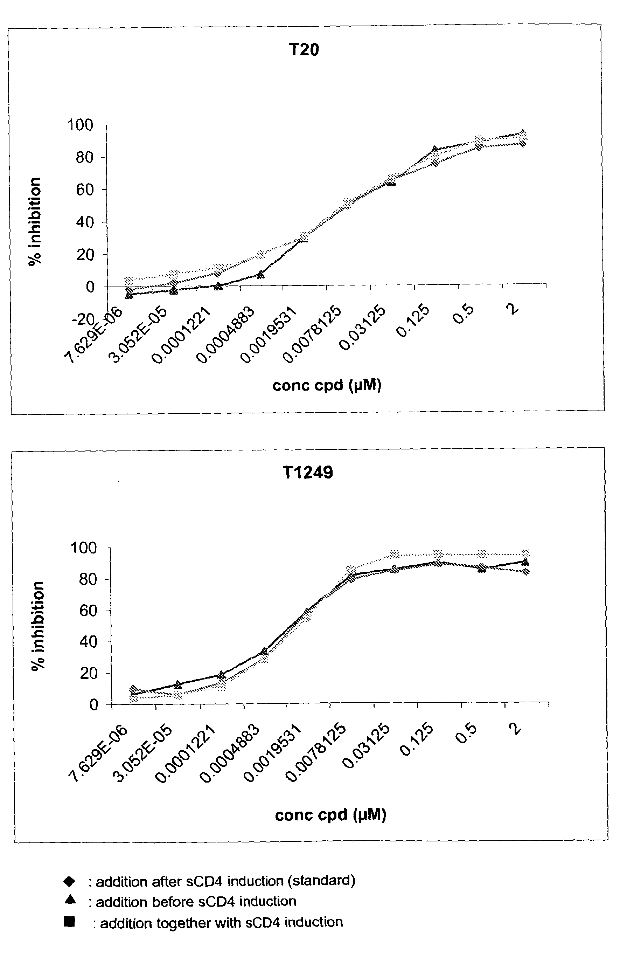

C34, T1249 and T20 did not show any difference between the three tested conditions (Fig. 1). In contrast, BMS-806 showed a large increase of sensitivity when the compound was added before or together with sCD4, compared with when BMS-806 was added after sCD4 induction.

### Example 7: Optimized protocols for flow cytometric based read-out and HCS native gp41

All previous examples were analyzed using flow cytometry. An optimized protocol for the flow cytometric based read-out, as well as the optimized protocol for the native gp41 assay performed on the high content screening (HCS) platform is provided hereafter.

### Material and methods

### Reagents

Human soluble CD4 was purchased from Protein sciences Corporation (Meridan, CT) (cat 3002) and Fluorescein isothiocyanate (FITC) labeled C34 peptide was synthesized according to standard protocols (Abgent, San Diego, CA). The C34 peptide covers a portion of the HR2 domain of gp41 of the HIV-1 envelope protein and the peptide was FITC labeled at the C terminus and is stored in DMSO. DRAQ5 was purchased from Biostatus (Leicestershire, United Kingdom). DRAQ5™ is a highly cell permeable DNA-interactive agent, with fluorescence signature extending into the infra-red region of the spectrum. It is a good choice for nuclear staining of live cells, as it does not require a UV laser source for excitation. DRAQS™ will work with most benchtop confocal systems and due to its far-red emission it is spectrally ideally compatible with GFP and FITC based fluors. DRAQ5™ is a pure synthetic compound with high affinity for DNA, it is stable at room temperature, under normal lighting conditions, and it is soluble in water at biologically compatible pH.

Non-peptide reference compounds were synthesized in-house and stored in DMSO. All reagents used for chemical synthesis, enzymatic reactions, and cell culture were purchased from commercial sources and used as such.

### Cell line

A Hut-IIIB persistently infected cell line was created by infecting T-cell lymphoma Hut78 cells with IIIB virus, followed by multiple cloning steps to obtain a stable cell line with high expression levels of gp120.

### Native assay in HCS

A serial dilution of test compounds, dissolved in DMSO, were diluted in culture medium (RPMI1640 with Ultraglutamin and Hepes w/o phenol red with 10% bovine calf serum (HyClone) and 20µg/l gentamycin) with a final DMSO concentration of 1% and added to 384 well Cell Carrier plates (Evotec Technologies). Hut-IIIB cells (15000 cells/ well) were added to these test plates and incubated for 30 min at room temperature. Next, equal volumes of sCD4 (final concentration of 250 nM) and FITC labeled C34 (final concentration of 0.5 nM) were mixed and transferred to the 384w test plates and incubated for 2 hours at room temperature. Finally, a solution of DRAQ 5 in PBS (final concentration of 5 µM) was added to the wells. After incubating the plates for 30' at room temperature, the test plates were measured using the Opera^{™} confocal microscope high content screening platform (Evotec Technologies). After every addition, mixing of the well content was achieved by placing the 384w plates on a 384w shaker (MixMate, Eppendorf) for 5 seconds at 2000rpm.

Image acquisition was performed using a 40x water immersion objective and simultaneous excitation of FITC and Draq5 fluorescence with a 488 and a 635 laser. Image analysis was performed using Acapella software (Evotec Technologies) and was based on an in-house written script, which consist of 3 key features: first, cells were identified by nuclei detection based on the Draq5 nuclear stain; second, cell membranes were identified and a ring structure surrounding the cell membrane was delineated; third, C34-FITC binding to the gp41 of the HUT-IIIB cells was determined by spot detection and analysis in the latter ring structure. Output parameters of the script included among others "mean (number of spots per cell)" and "mean (integrated spot signal)". EC₅₀ calculations were based on the spots per cell and EC₅₀ is defined as the concentration of compound achieving 50% inhibition of the C34-FITC binding as compared with positive controls. Furthermore, potential false positive results due to fluorescence or false negative results due to quenching can be assessed by comparing the inhibition curves generated by the two above mentioned output parameters. If both curves do not align than spectral properties of the tested compounds can influence the fluorescent based read out.

### Native assay in flow cytometric read-out

Experimental conditions were similar as for the HCS native gp41 assay with slight modifications: the assay was performed in transparent round-bottom 96 well plates using 75000 Hut-IIIb cells/well and final concentration of 0.25 nM C34-FITC was used. Flow cytometric based read-out allows both activity and toxicity assessment. Activity is based on the 'median fluorescence' as measured in FL1 and EC₅₀ is defined as the concentration of compound achieving 50% inhibition of the C34-FITC binding as compared with positive controls. Observed changes in the population distribution in an FSC-SSC dot plot assess toxicity.

### Results

Binding of C34-FITC to sCD4 induced Hut-IIIB cells results in a dotted fluorescent pattern on the membrane, whereas no fluorescent pattern is observed in the non-induced cells. Observed fluorescence in the non-induced Hut-IIIB cells is due to auto-fluorescence. Image analysis resulted in the identification of individual cells based on a nuclear stain and quantitative spot detection.

Based on the output parameter 'mean (number of spots/cell)' inhibition curves were generated and EC₅₀ values calculated for reference compounds (Table 4). These results clearly indicated that the native gp41 assay is biased towards fusion inhibitors, as T20, T1249 and C34 inhibit C34-FITC binding, whereas RT inhibitor EFV shows no activity. Observed activity in the HCS native gp41 assay is due to cytotoxicity of EFV at 200 µM.

Furthermore, due to the nature of the assay, inhibitors of CD4 binding to gp120 (e.g. BMS-806) are also active in the assay, but these can easily be discriminated from fusion inhibitors in down stream profiling assays. Moreover, the assay shows specificity as, a RSV specific fusion peptide, C39, shows no activity in the native gp41 assay.

Overall, a good correlation is observed for the flow cytometric- and Opera-based native gp41 assay results.

**Table 4. Activities of reference compounds tested in flow cytometric- and HCS-based native gp41 assay. Results are representative of 3 independent experiments.**

| Compound | Mechanism | EC₅₀ flow cytometry (µM) | EC₅₀ HCS (µM) |
|---|---|---|---|
| T20 | Fusion | 0.0025 | 0.0036 |
| T1249 | Fusion | Not tested | 0.00039 |
| C34 | Fusion | 0.0005 | 0.00024 |
| BMS-806 | CD4 -gp120 | 0.013 | 0.0043 |
| C39 | RSV fusion | >20 | >20 |
| EFV | RT | >200 | 104 |

## Claims

1. A in vitro method for measuring the interference of candidate compounds interfering in the formation of a complex between a C34 peptide derived from the heptad-repeat 2 (HR2) region of Human Immunodeficiency Virus (HIV) and the corresponding heptad-repeat 1 region of gp41 after induction with a human cellular soluble CD4 (sCD4) comprising:
contact cells, expressing native HIV-1 envelope spikes containing gp41, with human sCD4 to obtain sCD4 induced cells,
contact said induced cells with a candidate compound,
provide, optionally together with said candidate compound, a labeled C34 peptide of HIV-1 consisting essentially of the amino acid sequence SEQ ID NO:1 and
measure the interference of said compound with the complex formation between HR1 in the native spike and exogenously added labeled C34 by determining the % inhibition as a consequence of the presence or absence of said candidate compound respectively,
or
contact cells, expressing native HIV-1 envelope spikes containing gp41, with a candidate compound,
contact said cells with sCD4 to obtain sCD4 induced cells, provide, optionally together with said sCD4, a labeled C34 peptide of HIV-1 consisting essentially of the amino acid sequence SEQ ID NO:1 and
measure the interference of said compound with the complex formation between HR1 in the native spike and exogenously added labeled C34 by determining the % inhibition as a consequence of the presence or absence of said candidate compound respectively,
or
contact cells, expressing native HIV-1 envelope spikes containing gp41, with a candidate compound together with sCD4 to obtain sCD4 induced cells,
provide a labeled C34 peptide of HIV-1 consisting essentially of the amino acid sequence SEQ ID NO:1 and
measure the interference of said compound with the complex formation between HR1 in the native spike and exogenously added C34 by determining the % inhibition as a consequence of the presence or absence of said candidate compound respectively.

2. A method according to claim 1 wherein the cells are HIV-infected cells preferably wherein the HIV-infected cells are IIIB-infected HUT78 cells.

3. A method according to claim 1 or 2 wherein said label is an enzyme, green fluorescent protein or a mutant thereof, fluorescent substance, chemiluminescent substance or radioisotope or combinations thereof.

4. A method according to claim 3 wherein the label is FITC linked to C34.

5. A method according to claim 4 wherein FITC is linked to C34 by a linker with amino acid sequence GSSGGK (SEQ ID NO: 2).

6. A method according to any of the claims 3-5 wherein said measuring is performed by flow cytometry, fluorescence microscopy, fluorimetry, enzyme immunoassay, radiolabelling or chemiluminiscent techniques.

7. A method according claim 1 further comprising formulating the compound identified in a pharmaceutically acceptable form.

## Patentansprüche

1. In-Vitro-Verfahren zum Messen der Störung von Kandidatenverbindungen, die die Ausbildung eines Komplexes zwischen einem aus dem HR2(heptad-repeat 2)-Bereich des menschlichen Immunschwächevirus (HIV) stammenden C34-Peptid und dem entsprechenden HR1 (heptad-repeat 1)-Bereich von gp41 nach Induktion mit einem menschlichen zellulären löslichen CD4 (sCD4) stören, wobei man:
Zellen, die native HIV-1-Hüllen-Spikes mit gp41 exprimieren, mit menschlichem sCD4 in Kontakt bringt, so dass sCD4-induzierte Zellen erhalten werden,
die induzierten Zellen mit einer Kandidatenverbindung in Kontakt bringt,
gegebenenfalls zusammen mit der Kandidatenverbindung ein markiertes C34-Peptid von HIV-1, das im Wesentlichen aus der Aminosäuresequenz SEQ ID NO:1 besteht, bereitstellt und
die Störung der Komplexbildung zwischen HR1 in dem nativen Spike und exogen zugegebenem markiertem C34 durch die Verbindung misst, indem man die Hemmung in % als Folge des Vorliegens bzw. Fehlens der Kandidatenverbindung bestimmt,
oder
Zellen, die native HIV-1-Hüllen-Spikes mit gp41 exprimieren, mit einer Kandidatenverbindung in Kontakt bringt,
die Zellen mit sCD4 in Kontakt bringt, so dass sCD4-induzierte Zellen erhalten werden,
gegebenenfalls zusammen mit dem sCD4 ein markiertes C34-Peptid von HIV-1, das im Wesentlichen aus der Aminosäuresequenz SEQ ID NO:1 besteht, bereitstellt und
die Störung der Komplexbildung zwischen HR1 in dem nativen Spike und exogen zugegebenem markiertem C34 durch die Verbindung misst, indem man die Hemmung in % als Folge des Vorliegens bzw. Fehlens der Kandidatenverbindung bestimmt,
oder
Zellen, die native HIV-1-Hüllen-Spikes mit gp41 exprimieren, mit einer Kandidatenverbindung zusammen mit sCD4 in Kontakt bringt, so dass sCD4-induzierte Zellen erhalten werden,
ein markiertes C34-Peptid von HIV-1, das im Wesentlichen aus der Aminosäuresequenz SEQ ID NO:1 besteht, bereitstellt und
die Störung der Komplexbildung zwischen HR1 in dem nativen Spike und exogen zugegebenem C34 durch die Verbindung misst, indem man die Hemmung in % als Folge des Vorliegens bzw. Fehlens der Kandidatenverbindung bestimmt.

2. Verfahren nach Anspruch 1, wobei es sich bei den Zellen um HIV-infizierte Zellen handelt, wobei es sich bei den HIV-infizierten Zellen vorzugsweise um IIIB-infizierte HUT78-Zellen handelt.

3. Verfahren nach Anspruch 1 oder 2, wobei es sich bei der Markierung um ein Enzym, grünes Fluoreszenzprotein oder eine Mutante davon, einen Fluoreszenzstoff, einen Chemilumineszenzstoff oder ein Radioisotop oder Kombinationen davon handelt.

4. Verfahren nach Anspruch 3, wobei es sich bei der Markierung um mit C34 verknüpftes FITC handelt.

5. Verfahren nach Anspruch 4, wobei FITC mit C34 über einen Linker mit der Aminosäuresequenz GSSGGK (SEQ ID NO: 2) verknüpft ist.

6. Verfahren nach einem der Ansprüche 3-5, wobei die Messung mittels Durchflusszytometrie, Fluoreszenzmikroskopie, Fluorimetrie, Enzymimmuntest, radioaktiver Markierung oder Chemilumineszenztechniken erfolgt.

7. Verfahren nach Anspruch 1, bei dem man ferner die identifizierte Verbindung in einer pharmazeutisch unbedenklichen Form formuliert.

## Revendications

1. Procédé in vitro pour mesurer l'interférence de composés candidats qui interfèrent dans la formation d'un complexe entre un peptide C34 dérivé de la région de répétition en heptade 2 (HR2) du virus de l'immunodéficience humaine (VIH) et la région de répétition en heptade 1 correspondante du gp41 après induction avec un CD4 soluble (sCD4) cellulaire humain, comprenant :
la mise en contact de cellules, exprimant les spicules d'enveloppe du VIH-1 natifs, contenant le gp41, avec le sCD4 humain pour obtenir des cellules induites par le sCD4,
la mise en contact desdites cellules induites avec un composé candidat,
la mise à disposition, en option en même temps que ledit composé candidat, d'un peptide C34 marqué du VIH-1, consistant essentiellement en la séquence d'acides aminés SEQ ID N° 1, et
la mesure de l'interférence dudit composé avec la formation d'un complexe entre le HR1 dans le spicule natif et le CD34 marqué ajouté d'une manière exogène,
par détermination du pourcentage d'inhibition en conséquence respectivement de la présence ou de l'absence dudit composé candidat,
ou
la mise en contact de cellules, exprimant des spicules d'enveloppe du VIH-1 natifs, contenant le gp41, avec un composé candidat,
la mise en contact desdites cellules avec le sCD4 pour obtenir des cellules induites par le sCD4,
la mise à disposition, en option en même temps que ledit sCD4, d'un peptide C34 marqué du VIH-1,
consistant essentiellement en la séquence d'acides aminés SEQ ID N° 1, et
la mesure de l'interférence dudit composé avec la formation d'un complexe entre le HR1 dans le spicule natif et le C34 marqué ajouté d'une manière exogène,
par détermination du pourcentage d'inhibition en conséquence respectivement de la présence ou de l'absence dudit composé candidat,
ou
la mise en contact de cellules, exprimant des spicules d'enveloppe du VIH-1 natifs contenant le gp41, avec un composé candidat, en même temps que le sCD4, pour obtenir des cellules induites par le sCD4,
la mise à disposition d'un peptide C34 marqué du VIH-1,
consistant essentiellement en la séquence d'acides aminés SEQ ID N° 1 ; et
la mesure de l'interférence dudit composé avec la formation d'un complexe entre le HR1 dans le spicule natif et le C34 ajouté d'une manière exogène, par détermination du pourcentage d'inhibition en conséquence respectivement de la présence ou de l'absence dudit composé candidat.

2. Procédé selon la revendication 1, dans lequel les cellules sont des cellules infectées par le VIH, de préférence dans lequel les cellules infectées par le VIH sont des cellules HUT78 infectées par le IIIB.

3. Procédé selon la revendication 1 ou 2, dans lequel ledit marqueur est une enzyme, une protéine de fluorescence verte ou un mutant de cette dernière, une substance fluorescente, une substance chimioluminescente ou un radio-isotope ou des combinaisons de ceux-ci.

4. Procédé selon la revendication 3, dans lequel le marqueur est le FITC lié au C34.

5. Procédé selon la revendication 4, dans lequel le FITC est lié au C34 par un lieur ayant la séquence d'acides aminés GSSGGK (SEQ ID N° 2).

6. Procédé selon l'une quelconque des revendications 3 à 5, dans lequel ladite mesure est effectuée par des techniques de cytométrie en flux, de microscopie en fluorescence, de fluorimétrie, d'analyse enzymo-immunologique, de radiomarquage ou de chimioluminescence.

7. Procédé selon la revendication 1, qui comprend en outre la formulation du composé identifié, pour le mettre sous une forme acceptable d'un point de vue pharmaceutique.
